# EUROPEAN PATENT APPLICATION

(11) **EP 4 063 845 A1**
(43) Date of publication of application: **28.09.2022**
(21) Application number: 20890874.9
(22) Date of filing: 13.11.2020
(51) Int. Cl.: G01N 27/62, G01N 33/53, G01N 33/574, G01N 33/68

(54) **METHOD FOR DETECTING CANCER BONE METASTASIS AND DETECTION REAGENT**

(30) Priority: 22.11.2019 JP 2019211488
(71) Applicant: OSAKA UNIVERSITY, Suita-shi Osaka 565-0871 (JP); Tosoh Corporation, Yamaguchi 746-8501 (JP)
(72) Inventor: UEMURA, Motohide, Suita-shi, Osaka 565-0871 (JP); NONOMURA, Norio, Suita-shi, Osaka 565-0871 (JP); UJIKE Takeshi, Suita-shi, Osaka 565-0871 (JP); MYOBA, Shohei, Ayase-shi, Kanagawa 252-1123 (JP); OHTAKE, Norihisa, Ayase-shi, Kanagawa 252-1123 (JP)
(74) Representative: Novagraaf International SA
(86) International application number: PCT/JP2020/042388
(87) International publication number: WO 2021/100621

(57) **Abstract**

A problem to be addressed by the present invention is to provide: a method for detecting bone metastasis of cancer (excluding castration resistant prostate cancer) in a simple and highly accurate manner; and a reagent that can be used in the method. Bone metastasis of cancer (excluding castration resistant prostate cancer) is detected by measuring an intact growth and differentiation factor 15 (GDF15) propeptide level, a GDF15 propeptide fragment level, and the total of an intact GDF15 propeptide level and a GDF15 propeptide fragment level in a sample. The above described method for detecting bone metastasis of cancer includes a method for detecting bone metastasis of prostate cancer other than castration resistant prostate cancer, renal cancer, lung cancer, breast cancer, thyroid cancer, pancreatic cancer, bladder cancer, colon cancer, melanoma, myeloma, or lymphoma. Further, the reagent for detecting bone metastasis of cancer (excluding castration resistant prostate cancer) includes an antibody that specifically recognizes GDF15 propeptide.

## Description

### TECHNICAL FIELD

The present invention relates to a method and a reagent for detecting bone metastasis of cancer (excluding castration resistant prostate cancer (hereinafter referred to as "CRPC")) by measuring, as indicators, a propeptide of Growth and Differentiation Factor 15 (hereinafter also referred to as "GDF15") protein in blood, and a degradation product thereof.

### BACKGROUND ART

When cancer is advanced, cancer cells acquire metastatic ability, and cancer cells free from a cancer tissue are transported to each site in the body through vessels. Cancer is considered to be likely to metastasize to the lung and the liver with large blood flow volumes as well as the lymph nodes and the bone matrices rich in growth factors. Cancer that has metastasized to bone is called a metastatic bone tumor, and is known to result in bone pain, spinal cord compression, pathologic fracture, a surgery or radiation therapy for a bone metastatic focus, and/or a skeletal related event such as hypercalcaemia, and to adversely affect quality of life, and prognosis.

In the case of suspicion of bone metastasis, first, the assessment of the risk of fracture, and the selection of a therapeutic method are performed based on plain X-ray examination and/or computed tomography examination. Bone scintigraphy, ¹⁸F-fluorodeoxyglucose-positron emission tomography, positron emission tomography-computed tomography, and magnetic resonance imaging (hereinafter also referred to as "MRI") are highly useful for diagnosis of bone metastasis, and are also recommended in Practical Guideline of Bone Metastasis (2015) . Each method has both good points and bad points, and these image diagnoses are properly used depending on the type of cancer and the characteristics of bone metastasis, to detect a bone metastatic focus.

As *in vitro* diagnostic markers, bone metabolism markers listed in Table 1 have been reported to be able to be useful for monitoring in bone metastasis therapy. However, it has not been investigated that such bone metabolism markers are direct predictive factors for therapeutic effects; and use of the bone metabolism markers in daily medical practice has not been recommended in domestic and foreign guidelines.

Although being highly useful in diagnosis of bone metastasis, image diagnoses have problems that inter-reader variability occurs, radiation exposure is entailed, and devices (whole-body MRIs) do not come into wide use. Therefore, there is a demand for the discovery of a marker capable of detecting bone metastasis in a simple and highly accurate manner, and for the development of an examination method capable of easily detecting bone metastasis in a highly accurate manner.

**[Table 1]**

| | | |
|---|---|---|
| Bone Metabolism Marker | Bone Formation | Bone Specific Alkaline Phosphatase |
| | | Osteocalcin |
| | | Type I Collagen-N-Propeptide |
| | | Type I Collagen-C-Propeptide |
| | Bone Resorption | Type I Collagen Cross-Linked N-Telopeptides, Urine |
| | | Type I Collagen Cross-Linked C-Telopeptide |
| | | Type I Collagen C-Terminal Telopeptide |
| | | Deoxypyridinoline |
| | | Pyridinoline |
| | | Tartrate-resistant Phosphatase |

GDF15 is a protein which is identical to macrophage inhibitory cytokine 1 (MIC-1) and nonsteroidal anti-inflammatory drug-activated gene 1 (NAG-1), and belongs to the TGF-β family. GDF15 is expressed as prepro-GDF15, which contains a secretion signal and a propeptide, and then the secretion signal is cleaved off from the prepro-GDF15 to form pro-GDF15, which is then secreted outside the cell. Pro-GDF15 is stored in the extracellular matrix through the propeptide, and GDF15 forming a dimer is cleaved off from the propeptide by a furin-like protease to be released into blood (Non-patent Document 1). Full-length pro-GDF15 is reported to be fractionated into a molecular weight of about 40,000, and the mature body of GDF15 is reported to be fractionated into a molecular weight of about 15,000 (Non-patent Document 2) .

It has been confirmed that the level of the mature body of GDF15 in blood increases in various types of cancer, such as pancreatic cancer and colon cancer (Non-patent Documents 3 to 8). It has also been reported that GDF15 can be a potential indicator for prognostication associated with metastasis and malignancy in prostate cancer (Non-patent Documents 9 to 12).

It has been reported that the level of GDF15 propeptide in blood increases in CRPC, pancreatic cancer, colon cancer, lung cancer, breast cancer, esophageal cancer, stomach cancer, and small cell lung cancer (Patent Documents 1 to 2) . Patent Document 1 describes that a high correlation between intact GDF15 propeptide and indicators EOD and BSI for bone metastasis is not observed. On the other hand, however, Patent Document 1 also describes that malignant progression such as bone metastasis is observed in the case of the high level of intact GDF15 propeptide, suggesting that the level of intact GDF15 can be a potential marker for malignant progression of CRPC. Patent Document 1 provides mutually contradictory descriptions as described above. Thus, a relationship between GDF15 propeptide and bone metastasis has been unknown.

GDF15 propeptide (hereinafter also referred to as "GDPP") is a polypeptide of 165 residues located on the N-terminal side of pro-GDF15. More specifically, the GDF15 propeptide in the present specification contains at least an amino acid sequence from the leucine at the 30th residue to the arginine at the 194th residue, which is a sequence following the region of the signal peptide from the initiating methionine to the alanine at the 29th residue, in an amino acid sequence (SEQ ID NO:2) based on cDNA (GeneBank Accession No.: NM_004864) of human GDF15 shown in SEQ ID NO:1, or contains an amino acid sequence having an identity of not less than 80% to the above described sequence.

### PRIOR ART DOCUMENTS

### Patent Documents

Patent Document 1: WO 2017/150314
Patent Document 2: JP 2019-045486 A

### Non-patent Documents

Non-patent Document 1: Prostate Cancer Prostatic Dis. 2012; 15 (4): 320-328
Non-patent Document 2: Cancer Res. 2005; 65 (6): 2330-2336
Non-patent Document 3: BMC Cancer. 2014; 14: 578-588
Non-patent Document 4: Biochemical Pharmacology. 2013; 85: 597-606
Non-patent Document 5: Clin Cancer Res. 2009; 15 (21): 6658-6664
Non-patent Document 6: Clin Cancer Res. 2011; 17: 4825-4833
Non-patent Document 7: Clin Cancer Res. 2003; 9: 2642-2650
Non-patent Document 8: Clin Cancer Res. 2006; 12: 442-446
Non-patent Document 9: Cancer Epidemiol Biomarders Prev. 2007; 16 (3): 532-537
Non-patent Document 10: Anticancer Research. 2016; 36: 1973-1978
Non-patent Document 11: Anticancer Research. 2017; 37 (3): 1501-1505
Non-patent Document 12: Technology in Cancer Research & Treatment. 17: 1-7

### SUMMARY OF THE INVENTION

### PROBLEMS TO BE SOLVED BY THE INVENTION

An object of the present invention is to provide a method for detecting bone metastasis of cancer in a simple and highly accurate manner, and a reagent that can be used in the method.

### MEANS FOR SOLVING THE PROBLEMS

The present inventors have made intensive studies in order to solve the above-mentioned problems. As a result, the present inventors have found out that, in an immunoassay using an antibody that recognizes GDF15 propeptide, an increase in the level of GDF15 propeptide in blood is observed in samples from cases of bone metastasis of prostate cancer (excluding CRPC), renal cancer, lung cancer, breast cancer, thyroid cancer, pancreatic cancer, bladder cancer, colon cancer, melanoma, myeloma, or lymphoma, as compared to that in healthy samples and samples from cases of no bone metastasis of all the types of the cancer. Based on these findings, the present inventors discovered that GDF15 propeptide can be a potential marker for detecting bone metastasis of cancer (excluding CRPC), thereby completing the present invention.

That is, the present invention is as follows.
[1] A method for detecting bone metastasis of cancer (excluding castration resistant prostate cancer), the method including measuring an intact growth and differentiation factor 15 (GDF15) propeptide level in a sample.
[2] A method for detecting bone metastasis of cancer (excluding castration resistant prostate cancer), the method including measuring a GDF15 propeptide fragment level in a sample.
[3] A method for detecting bone metastasis of cancer (excluding castration resistant prostate cancer), the method including measuring a total of an intact GDF15 propeptide level and a GDF15 propeptide fragment level in a sample.
[4] The method according to [2] or [3], wherein the GDF15 propeptide fragment(s) include(s) the following GDF15 propeptide fragment(s) (A) and/or (B):
   (A) a GDF15 propeptide fragment having the following properties:
      contains an amino acid sequence from the lysine at the 58th residue to at least the aspartic acid at the 167th residue in the GDF15 amino acid sequence of SEQ ID NO:2, or a sequence having an identity of not less than 80% thereto; and
   (B) a GDF15 propeptide fragment having the following properties:
      contains an amino acid sequence from the glutamic acid at the 74th residue to at least the aspartic acid at the 167th residue in the GDF15 amino acid sequence of SEQ ID NO:2, or a sequence having an identity of not less than 80% thereto.
[5] The method according to any one of [1] to [4], wherein bone metastasis of prostate cancer other than castration resistant prostate cancer, renal cancer, lung cancer, breast cancer, thyroid cancer, pancreatic cancer, bladder cancer, colon cancer, melanoma, myeloma, or lymphoma is detected.
[6] The method according to any one of [1] to [5], wherein the measurement is carried out by an antigen-antibody reaction using an antibody that recognizes GDF15 propeptide.
[7] The method according to any one of [1] to [5], wherein the measurement is carried out using mass spectrometry.
[8] A reagent for detecting bone metastasis of cancer (excluding castration resistant prostate cancer), the reagent including an antibody that specifically recognizes GDF15 propeptide.

### EFFECT OF THE INVENTION

The present invention provides a method for detecting bone metastasis of cancer (excluding CRPC) in a simple and highly accurate manner, and a reagent that can be used in the method.

The reagent according to the present invention is used for detecting GDF15 propeptide, and GDF15 is one belonging to the TGF-β family. Therefore, the degree of osteoclast activation caused by cytokine released from a cancer cell may be reflected. In such a case, it is presumed that the therapeutic effects of existing bone modifying agents are reflected. Accordingly, the reagent according to the present invention can also be used as a companion diagnostic agent in the treatment of bone metastasis of cancer (excluding CRPC).

### BRIEF DESCRIPTION OF THE DRAWINGS

FIG. 1 is a diagram showing box plots of measured values of various markers in the serums of benign samples (negative biopsy), prostate cancer (without bone metastasis) samples, and samples of metastatic bone tumor of prostate cancer.
FIG. 2 is a diagram showing the results of receiver operating characteristic (ROC) curve analysis of various markers in serums of the prostate cancer (without bone metastasis) samples and the samples of metastatic bone tumor of prostate cancer, or the benign samples (negative biopsy) and the samples of metastatic bone tumor of prostate cancer.
FIG. 3 is a diagram showing the results of ROC curve analysis of various markers using the simultaneously sampled serums and plasmas of the prostate cancer (without bone metastasis) samples and the samples of metastatic bone tumor of prostate cancer.
FIG. 4 is a diagram showing box plots of various markers in the serums of samples of renal cancer (without bone metastasis) and metastatic bone tumor of renal cancer.
FIG. 5 is a diagram showing the results of ROC curve analysis of the various markers in the serums of the samples of renal cancer (without bone metastasis) and metastatic bone tumor of renal cancer.
FIG. 6 is a diagram showing box plots of various markers in the plasma of a normal sample, or a sample of metastatic bone tumor of lung cancer or metastatic bone tumor of breast cancer.
FIG. 7 is a diagram showing the results of ROC curve analysis of the various markers in the plasma of the normal sample, or the sample of metastatic bone tumor of lung cancer or metastatic bone tumor of breast cancer.
FIG. 8 is a diagram showing box plots of various markers in the serums of cases without bone metastasis and cases with metastatic bone tumor in CRPC.
FIG. 9 is a diagram showing box plots of various markers in the serums of cases without bone metastasis and cases with metastatic bone tumor in prostate cancer which is not CRPC.
FIG. 10 is a diagram showing the results of ROC curve analysis of the various markers in the serums of the cases without bone metastasis and the cases with metastatic bone tumor in prostate cancer which is not CRPC.
FIG. 11 is a diagram showing box plots of various markers based on samples of lung cancer (without metastasis), lung cancer (with metastasis to any site other than bone), and metastatic bone tumor of lung cancer.
FIG. 12 is a diagram showing the results of ROC curve analysis of various markers based on samples of cases without bone metastasis and cases with metastatic bone tumor in lung cancer.
FIG. 13 is a diagram showing box plots of various markers based on samples of breast cancer (without metastasis), breast cancer (with metastasis to any site other than bone), and metastatic bone tumor of breast cancer.
FIG. 14 is a diagram showing the results of ROC curve analysis of various markers based on samples of cases without bone metastasis and cases with metastatic bone tumor in breast cancer.
FIG. 15 is a diagram showing box plots of various markers in the plasmas of normal samples, and samples of metastatic bone tumor of thyroid cancer, pancreatic cancer, bladder cancer, colon cancer, melanoma, myeloma, and lymphoma.

### MODE FOR CARRYING OUT THE INVENTION

### <1> Method for Detecting Bone Metastasis of Cancer (Excluding CRPC) of Present Invention

The first aspect of the present invention is a method for detecting bone metastasis of cancer (excluding CRPC), and the method includes measuring the level of GDF15 propeptide in a sample. This is a method based on the fact that GDF15 propeptide is characteristically present in a biological sample such as blood of a patient with bone metastasis of cancer (excluding CRPC), as compared to samples of a healthy individual and a patient with cancer without bone metastasis . The level of GDF15 propeptide in a sample is usually measured *in vitro.*

This method enables, as will be shown in Examples to be described later, to detect bone metastasis of cancer (excluding CRPC), with a higher sensitivity and specificity, as compared to the case of measuring a conventionally known bone metabolism marker (ALP).

The method of the present invention includes steps up until a step of detecting bone metastasis of cancer (excluding CRPC), and does not include final determination action for diagnosis of the bone metastasis. A doctor diagnoses bone metastasis and creates a treatment plan with reference to detection results and the like obtained in the method of the present invention.

Commonly, a subject (test animal) in which bone metastasis is intended to be detected is a human.

The GDF15 propeptide to be measured in the present aspect includes: intact GDF15 propeptide (hereinafter, also referred to as "iGDPP") which includes the amino acid sequence from the leucine at the 30th residue to the arginine at the 194th residue in the GDF15 amino acid sequence of SEQ ID NO:2, or an amino acid sequence having an identity of not less than 80% to the sequence described above; and GDF15 propeptide fragments. The intact GDF15 propeptide refers to the GDF15 propeptide that has not been subjected to processing (that has not been degraded). The GDF15 propeptide fragments include dNT57-GDPP (peptide including an amino acid sequence from the 58th residue to the 167th residue of the amino acid sequence of SEQ ID NO:2, or an amino acid sequence having an identity of not less than 80% to the sequence described above), dNT73-GDPP (peptide including an amino acid sequence from the 74th residue to the 167th residue of the amino acid sequence of SEQ ID NO:2, or an amino acid sequence having an identity of not less than 80% to the sequence described above), and other peptide fragments. The other peptide fragments are not particularly limited as long as being peptide fragments formed after processing of GDF15 propeptide, and are preferably peptide including a sequence partly included in the amino acid sequence of SEQ ID NO:2, or an amino acid sequence having an identity of not less than 80% to the sequence described above.

In the detection method according to the present invention, the method for measuring the GDF15 propeptide level is not particularly limited. Examples of the method include a method utilizing an antigen-antibody reaction using an antibody that specifically recognizes GDF15 propeptide, and a method utilizing mass spectrometry.

Specific examples of the method utilizing an antigen-antibody reaction using an antibody that specifically recognizes GDF15 propeptide include the following.
(a) A competition method in which a labeled measuring object and an antibody that specifically recognizes the measuring object are used, and which utilizes the competitive binding of the labeled measuring object and the measuring object contained in the sample to the antibody.
(b) A method using surface plasmon resonance, in which the sample is brought into contact with a chip on which an antibody that specifically recognizes the measuring object is immobilized, and a signal dependent on the binding of the antibody to the measuring object is detected.
(c) A fluorescence polarization immunoassay in which an antibody that specifically recognizes a fluorescently-labeled measuring object is used, and which utilizes the phenomenon that the binding of the antibody to the measuring object causes an increase in the degree of fluorescence polarization.
(d) A sandwich method in which two types of antibodies (one of which is a labeled antibody) that specifically recognize different epitopes on the measuring object are used, so as to allow the formation of a complex of three molecules, namely, a complex of the two antibodies and the measuring object.
(e) A method in which the measuring object in the sample is concentrated by an antibody that specifically recognizes the measuring object, as a pretreatment, and then the polypeptide in the bound protein is detected using a mass spectrometer or the like.

Although the methods (d) and (e) are simple and highly versatile, the method (d) is more preferred for processing a large number of samples, since the technologies related to the reagents and the devices used in this method have been sufficiently established.

As the antibody that specifically recognizes GDF15 propeptide, an antibody that specifically recognizes the N-terminal region of GDF15 propeptide, for example, an antibody that binds to an antigenic determinant in the region from the leucine at the 30th residue to the arginine at the 57th residue in SEQ ID NO:2 can be preferably used for the measurement of the iGDPP level. Further, an antibody that specifically recognizes the C-terminal region of GDF propeptide, for example, an antibody that binds to an antigenic determinant in the region from the glutamic acid at the 74th residue to the arginine at the 196th residue in SEQ ID NO:2 can be preferably used for the measurement of the total of the iGDPP level and the GDPP fragment level (total GDPP; hereinafter also referred to as "tGDPP").

The antibody that specifically recognizes GDF15 propeptide can be obtained by immunizing an animal using as an immunogen, for example, GDF15 propeptide itself, an oligopeptide composed of a partial region of GDF15 propeptide, or a polynucleotide encoding intact pro-GDF15 protein or a partial region thereof.

The animal to be used for the immunization is not particularly limited as long as the animal has ability to produce antibodies. The animal may be a mammal normally used for immunization, such as mouse, rat, or rabbit, or may be a bird such as chicken.

In cases where GDF15 propeptide itself or an oligopeptide composed of a partial region of GDF15 propeptide is used as the immunogen, the structure of the protein or the oligopeptide may change during the preparation process thereof. Therefore, the resulting antibody may not have a high specificity or binding capacity to the desired antigen, in some cases, possibly resulting in a failure to accurately quantify the level of GDF15 propeptide contained in the sample. On the other hand, in cases where an expression vector containing a polynucleotide encoding intact pro-GDF15 protein or a partial region thereof is used as the immunogen, the intact GDF15 propeptide protein or the partial region thereof introduced is expressed as it is, without undergoing a structural change in the body of the immunized animal. Therefore, an antibody having a high specificity and binding capacity (namely, a high affinity) to the GDF15 propeptide in the sample can be obtained, which is preferred.

The antibody that specifically recognizes GDF15 propeptide may be either a monoclonal antibody or a polyclonal antibody. The antibody is preferably a monoclonal antibody.

The establishment of a hybridoma cell that produces an antibody that specifically recognizes GDF15 propeptide can be carried out by a method selected as appropriate from methods whose techniques have been established. For example, a hybridoma cell that produces a monoclonal antibody that specifically recognizes GDF15 propeptide can be established by collecting B cells from an animal immunized by the above method, fusing the B cells with myeloma cells electrically or in the presence of polyethylene glycol, selecting a hybridoma cell that produces a desired antibody using HAT medium, and preparing the selected hybridoma cell into a monoclone by the limiting dilution method.

The selection of the antibody that specifically recognizes GDF15 propeptide, for example, the monoclonal antibody that specifically recognizes GDF15 propeptide, to be used in the method for detecting bone metastasis of cancer (excluding CRPC) according to the present invention, can be carried out based on affinity to a GPI (glycosyl phosphatidyl inositol)-anchor type GDF15 propeptide or secretory GDF15 propeptide derived from a host expression system.

The host is not particularly limited, and can be selected as appropriate from cells of microorganisms such as *E. coli* and yeast, insect cells and animal cells that are usually used for protein expression by those skilled in the art. The host is preferably a mammalian cell, since it enables the expression of a protein having a structure similar to that of natural GDF15 propeptide by post-translational modification such as disulfide bonding or glycosylation. Examples of the mammalian cell include the human embryonic kidney (HEK) -derived 293T cell line, monkey kidney COS7 cell line, Chinese hamster ovary (CHO) cells, and cancer cells isolated from humans, which are conventionally used.

The purification of the antibody to be used in the method for detecting bone metastasis of cancer (excluding CRPC) according to the present invention can be carried out by a method selected as appropriate from methods whose techniques have been established. For example, after culturing hybridoma cells which are established by the above method and which produce an antibody, the culture supernatant may be collected, and the antibody may be concentrated, if necessary, by ammonium sulfate precipitation. Thereafter, affinity chromatography using a carrier to which Protein A, Protein G, Protein L or the like is immobilized, and/or ion-exchange chromatography can be carried out to achieve the purification of the antibody.

The labeled antibody to be used for the antigen-antibody reaction in the sandwich method described above can be prepared by labeling an antibody purified by the above method with, for example, an enzyme such as peroxidase or alkaline phosphatase. The labeling may also be carried out using a method whose technique has been sufficiently established.

The method for detecting GDF15 propeptide utilizing mass spectrometry, in the detection method according to the present invention, will be specifically described below.

In the case of using blood as a sample, it is preferred that proteins such as albumin, immunoglobulin and transferrin, which are contained in large amounts in blood, be removed as a pretreatment step, using Agilent Human 14 or the like, followed by further fractionation by ion exchange, gel filtration, reverse-phase HPLC, and/or the like.

The measurement can be carried out by tandem mass spectrometry (MS/MS), liquid chromatography-tandem mass spectrometry (LC/MS/MS), matrix assisted laser desorption ionization time-of-flight mass spectrometry (MALDI-TOF/MS), surface enhanced laser desorption ionization mass spectrometry (SELDI-MS), or the like.

In the detection method according to the present invention, it is preferred to determine that bone metastasis of cancer (excluding CRPC) is detected, when the GDF15 propeptide level obtained by the measurement is higher than a reference value (cutoff value) calculated from a control.

The GDF15 propeptide level to be used for the determination may be either a measured value or a converted concentration value. The converted concentration value refers to a value converted from a measured value based on a calibration curve prepared using GDF15 propeptide as a standard sample. The concentration of the standard sample may be determined as a value converted from a measured value based on a calibration curve of a standard peptide, prepared using mass spectrometry.

The reference value (cutoff value) may be set as appropriate to a measured value which provides an optimum sensitivity and specificity, by measuring samples of patients with cancer without bone metastasis and samples of patients with cancer (excluding CRPC) with bone metastasis, and then carrying out the receiver operating characteristic (ROC) curve analysis.

The method for detecting bone metastasis according to the present invention can be applied to a method for treating metastatic bone tumor. In other words, the present invention provides a method for treating metastatic bone tumor in a patient, the method including the steps of:
(i) identifying a patient in which the measured value of a GDF15 propeptide level is more than a reference value set in advance; and
(ii) performing treatment of the identified patient.

In the identification of the step (i), the measurement of the GDF15 propeptide level may be performed either using an antibody that specifically recognizes GDF15 propeptide or using mass spectrometry.

Examples of the treatment of the step (ii) include, but are not particularly limited to, surgical treatment, drug treatment, and radiation therapy.

### <2> Reagent for Detecting Bone Metastasis of Cancer (Excluding CRPC) according to Present Invention

The second aspect of the present invention is a reagent for detecting bone metastasis of cancer (excluding CRPC), and the reagent includes an antibody that recognizes GDF15 propeptide. The antibody is usually an antibody that binds to an antigenic determinant in the region from the leucine at the 30th residue to the arginine at the 196th residue in the pro-GDF15 represented by SEQ ID NO:2.

The GDF15 propeptide to be recognized by an antibody in the present aspect includes intact GDF15 propeptide and/or GDF15 propeptide fragments. The GDF15 propeptide fragments include dNT57-GDPP, dNT73-GDPP, and other peptide fragments. The descriptions of the propeptide and the propeptide fragments are similar to those of the first aspect described above.

In cases where the reagent according to the present invention is used in the sandwich method described above, the reagent needs to contain, as the antibody, two types of antibodies specific for different epitopes.

The detection reagent according to the present invention may further contain a detection reagent for detecting a bone metabolism marker, which detection reagent contains an antibody that specifically recognizes the bone metabolism marker. Examples of the bone metabolism marker include those shown in Table 1.

The antibody contained in the reagent according to the present invention may be an antibody itself, a labeled antibody, or an antibody immobilized on a solid phase.

The reagent according to the present invention will now be specifically described, regarding the case in which the reagent is used in a two-step sandwich method, which is one embodiment of the sandwich method described above. However, the present invention is not limited thereto.

The reagent according to the present invention can be prepared by the method described in the following (I) to (III).
(I) Of the two types of antibodies (hereinafter referred to as "Antibody 1" and "Antibody 2") that specifically recognize different epitopes on GDF15 propeptide and that are used in the sandwich method, Antibody 1 is first bound to a carrier capable of B/F (Bound/Free) separation, such as an immunoplate or magnetic particles. The Antibody 1 may be physically bound to the carrier utilizing hydrophobic bonding, or may be chemically bound thereto using, for example, a linker reagent capable of cross-linking two substances to each other.
(II) After binding the Antibody 1 to the carrier, the surface of the carrier is subjected to a blocking treatment using bovine serum albumin, skim milk, a commercially available immunoassay blocking agent or the like, for preventing non-specific binding, to provide a primary reagent.
(III) The other antibody, Antibody 2, is then labeled, and a solution containing the resulting labeled antibody is prepared as a secondary reagent. Preferred examples of the substance to be used for labeling the Antibody 2 include enzymes such as peroxidase and alkaline phosphatase; substances detectable by detection devices, such as fluorescent substances, chemiluminescent substances and radioisotopes; and substances to which another molecule specifically binds, such as biotin, to which avidin specifically binds. Preferred examples of the solution to be used for the secondary reagent include buffers which allow an antigen-antibody reaction to proceed favorably, such as phosphate buffer and Tris-HCl buffer.

The thus prepared reagent according to the present invention may be freeze-dried, if necessary.

In the case of performing a one-step sandwich method, the binding of Antibody 1 to the carrier and subsequent blocking treatment can be carried out in the same manner as in (I) and (II) to prepare an antibody-immobilized carrier, and a buffer containing a labeled Antibody 2 can be further added to the antibody-immobilized carrier, to prepare a reagent.

For the detection and measurement of GDF15 propeptide by a two-step sandwich method, using the reagent obtained by the method described above, the method described in the following (IV) to (VI) can be carried out. (IV) The primary reagent prepared in (II) is brought into contact with a sample for a predetermined period of time at a constant temperature . The reaction can be carried out under the conditions of a temperature within the range of from 4°C to 40°C for 5 minutes to 180 minutes.

(V) Unreacted substances are removed by B/F separation, and then the secondary reagent prepared in (III) is brought into contact with the reaction product for a predetermined period of time at a constant temperature, to allow the formation of a sandwich complex. The reaction can be carried out under the conditions of a temperature within the range of from 4°C to 40°C for 5 minutes to 180 minutes.

(VI) Unreacted substances are removed by B/F separation, and the labeling substance of the labeled antibody is quantified. Based on a calibration curve prepared using a GDF15 propeptide solution having a known concentration as a standard, the concentration of human GDF15 propeptide in the sample is quantified.

The amount of each reagent component, such as the antibody contained in the detection reagent, can be set as appropriate depending on the conditions such as the amount of the sample, the type of the sample, the type of the reagent, and the detection method. Specifically, for example, in cases where the GDF15 propeptide level is measured as described below by a sandwich method using 50 µL of 2.5-fold diluted serum or plasma as a sample, the amount of the antibody to be bound to the carrier may be from 100 ng to 1,000 µg, and the amount of the labeled antibody may be from 2 ng to 20 µg in a reaction system in which 50 µL of the sample is reacted with the antibodies.

The reagent for detecting bone metastasis of cancer (excluding CRPC) according to the present invention is applicable to either manual detection or detection using an automatic immunodiagnostic apparatus. In particular, the detection using an automatic immunodiagnostic apparatus is preferred, since it enables the detection of bone metastasis without being affected by endogenous measurement-interference factors and competing enzymes contained in the sample, and also enables the quantification of the concentrations of GDF15 propeptide and a bone metabolism marker in the sample, in a short period of time.

Another side of the second aspect of the present invention is use of an antibody that specifically recognizes GDF15 propeptide, in production of a reagent for detecting bone metastasis of cancer (excluding CRPC).

Another side of the second aspect of the present invention is use of an antibody that specifically recognizes GDF15 propeptide, in detection of bone metastasis of cancer (excluding CRPC).

The sample (test sample) to be measured by the method for detecting bone metastasis of cancer (excluding CRPC) according to the present invention and the detection reagent according to the present invention include is commonly a sample collected from a patient with cancer. More specific examples of the sample include, but are not limited to, samples collected from patients with prostate cancer other than castration resistant prostate cancer, renal cancer, lung cancer, breast cancer, thyroid cancer, pancreatic cancer, bladder cancer, colon cancer, melanoma, myeloma, or lymphoma. Of these, samples collected from patients with prostate cancer other than castration resistant prostate cancer, renal cancer, lung cancer, or breast cancer are preferred, and samples collected from patients with renal cancer, lung cancer, and breast cancer are still more preferred.

In other words, the bone metastasis to be preferably detected in the method according to the present invention is bone metastasis of prostate cancer other than castration resistant prostate cancer, renal cancer, lung cancer, breast cancer, thyroid cancer, pancreatic cancer, bladder cancer, colon cancer, melanoma, myeloma, or lymphoma.

Examples of the sample according to the present invention include: blood components such as whole blood, blood cells, serum, and plasma; extracts from cells and tissues; urine; and cerebrospinal fluid. A blood component or a body fluid such as urine is preferably used as the sample, since it allows for detecting bone metastasis of cancer (excluding CRPC) in a simple and noninvasive manner. From the viewpoint of the ease of sample collection and versatility for other test items, the use of a blood component as the sample is particularly preferred.

When a sample is used for detection in the present invention, a sample such as such blood collected from a patient may be used without being processed, or the sample subjected to processing such as dilution or addition of an anticoagulant as appropriate may be used. When the sample is diluted, the dilution rate of the sample may be selected as appropriate from no dilution to 100-fold dilution depending on the type and the conditions of the sample used. In the case of serum or plasma, for example, 50 µL of a 2.5-fold diluted sample can be used.

Timing at which the sample according to the present invention is collected is not particularly limited as long as being any timing after diagnosis of cancer. The timing may be in the early or advanced stage of cancer, may be after any treatment such as surgical treatment, drug treatment, or radiation therapy, or may be in a follow-up period after any medical treatment. A sample collected in any stage can be used in the method according to the present invention.

### EXAMPLES

Examples will be shown below for specifically describing the present invention. The following Examples are provided for illustrating the present invention, and the present invention is in no way limited to these Examples.

### <Example 1> Preparation of GDF15 Propeptide Assay Reagents

Two types of GDPP assay reagents were prepared with reference to the description of Patent Document 2, and used for assay. One of the reagents is based on the combination of an antibody (TS-GDPP02) that recognizes the N-terminal region of GDPP and an antibody (TS-GDPP04) that recognizes the C-terminal region of GDPP, and this reagent detects intact GDPP (iGDPP) . The other reagent is based on the combination of antibodies (TS-GDPP04 and TS-GDPP08) each of which recognizes the C-terminal region of GDPP, and this reagent detects both iGDPP and GDF15 propeptide fragments (including dNT57-GDPP and dNT73-GDPP) of which the N-terminal regions are deleted. The value detected by the latter reagent is defined as the total GDPP (tGDPP).

### <Example 2> Performance of Determination of Metastatic Bone Tumor in Prostate Cancer Serum Samples

The details of the serum sample panel (total of 54 cases) used in the present Example are shown in Table 2. All the samples were collected in the same protocol in the Department of Urology at Osaka University Hospital. The samples were provided after obtainment of informed consent and approval by the Clinical Research Review Committee in Osaka University.

**[Table 2]**

| | cases |
|---|---|
| Benign (Negative Biopsy) | 14 |
| Prostate Cancer (Without Bone Metastasis) | 22 |
| Metastatic Bone Tumor of Prostate Cancer | 18 |

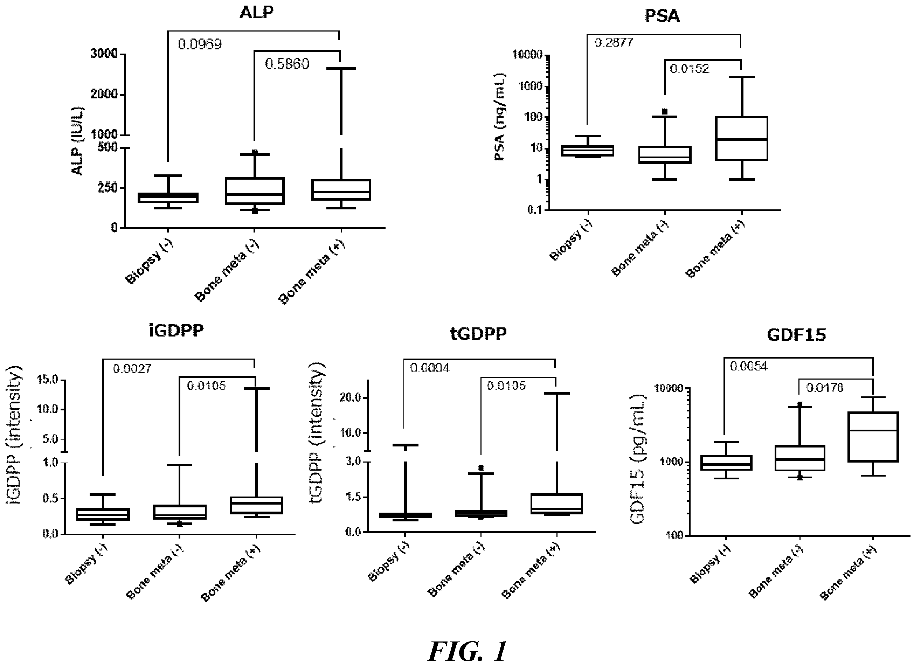

In this examination, alkaline phosphatase (ALP), prostate specific antigen (PSA), iGDPP, tGDPP, and GDF15 were assayed, and their performances of determination of metastatic bone tumor in prostate cancer were compared. The measured values of PSA, iGDPP, and tGDPP were calculated using a dedicated assay reagent and a fully automatic enzyme immunoassay apparatus AIA-600II (manufactured by Tosoh Corporation), and the measured value of GDF15 was calculated using a commercially available ELISA kit (R & D Corporation) . For ALP, reference to measured values just after blood sampling in medical service under health insurance was performed. Box plots of the various measured values are shown in FIG. 1, and significant test results as determined by Mann Whitney's U test are shown in Table 3.

**[Table 3]**

| | | P Values of Mann Whitney's U Test | |
|---|---|---|---|
| | | Prostate Cancer (Without Bone Metastasis) (22 Cases) Vs Metastatic Bone Tumor of Prostate Cancer (18 Cases) | Benign Samples (Negative Biopsy) (14 Cases) Vs Metastatic Bone Tumor of Prostate Cancer (18 Cases) |
| ALP | Serum | 0.586 | 0.097 |
| iGDPP | | 0.011 | 0.003 |
| tGDPP | | 0.011 | < 0.001 |
| GDF15 | | 0.018 | 0.005 |
| PSA | | 0.015 | 0.288 |

For the performance of determination of the metastatic bone tumor in the prostate cancer, a significant difference was observed in each of the four markers excluding ALP, and the highest P value was shown in iGDPP and tGDPP. For the performance of determination between benign cases with negative biopsy and the metastatic bone tumor of the prostate cancer, a significant difference was observed in each of iGDPP, tGDPP, and GDF15, and the highest P value was shown in tGDPP.

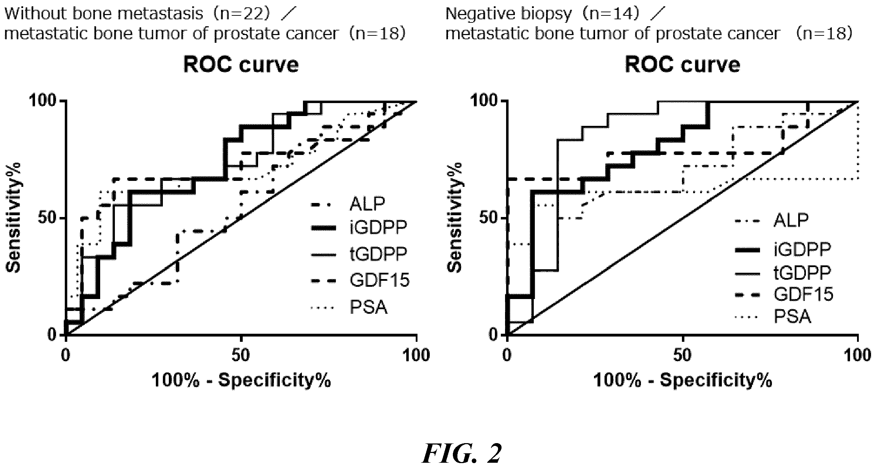

Then, examination of the performance of determination of the metastatic bone tumor in the prostate cancer was carried out by receiver operating characteristic (ROC) curve analysis. The analysis results are shown in FIG. 2, and the values of AUC (Area Under the Curve; the area under the ROC curve) are shown in Table 4.

**[Table 4]**

| | | Area Under the Curve (AUC) | |
|---|---|---|---|
| | | Prostate Cancer (Without Bone Metastasis) (22 Cases) Vs Metastatic Bone Tumor of Prostate Cancer (18 Cases) | Benign Samples (Negative Biopsy) (14 Cases) Vs Metastatic Bone Tumor of Prostate Cancer (18 Cases) |
| ALP | Serum | 0.552 | 0.675 |
| iGDPP | | 0.735 | 0.806 |
| tGDPP | | 0.735 | 0.853 |
| GDF15 | | 0.718 | 0.786 |
| PSA | | 0.708 | 0.613 |

For each of the performance of determination of the metastatic bone tumor in the prostate cancer and the performance of determination between the benign cases with negative biopsy and the metastatic bone tumor of the prostate cancer, iGDPP and tGDPP were shown to be superior to the other markers including ALP.

### <Example 3> Performance of Determination of Metastatic Bone Tumor Using Samples Obtained by Simultaneously Sampling Serum/Plasma in Prostate Cancer

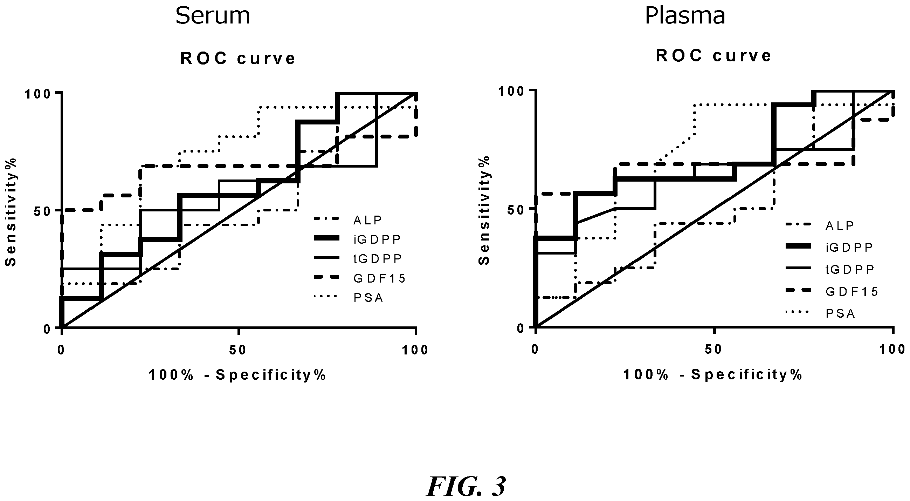

Of the prostate cancer serum samples analyzed in Example 2, the samples of cases of which the serums and plasmas were simultaneously sampled (9 cases with prostate cancer (without bone metastasis) and 16 cases with metastatic bone tumor of prostate cancer) were used to compare the diagnosis performances of various markers between serum and plasma by Mann Whitney's U test or ROC analysis. The P values and AUC values of the U test are shown in Table 5, and the ROC analysis is shown in FIG. 3.

**[Table 5]**

| | | Prostate Cancer (Without Bone Metastasis) (9 Cases) VS Metastatic Bone Tumor of Prostate Cancer (16 Cases) | |
|---|---|---|---|
| | | P values of Mann Whitney' s U test | Area Under the Curve (AUC) |
| iGDPP | Serum | 0.411 | 0.604 |
| | Plasma | 0.084 | 0.715 |
| tGDPP | Serum | 0.588 | 0.569 |
| | Plasma | 0.257 | 0.642 |
| GDF15 | Serum | 0.152 | 0.681 |
| | Plasma | 0.152 | 0.681 |
| PSA | Serum | 0.055 | 0.736 |
| | Plasma | 0.152 | 0.729 |

For the diagnosis performances of GDF15 and PSA, no great difference was observed between serum and plasma. On the other hand, it was revealed that the diagnosis performance of iGDPP was greatly improved in plasma, as compared to serum. Previous studies have shown that GDPP tends to be easily degraded by protease and the like, and it was suggested that use of plasma is preferable for detecting iGDPP.

### <Example 4> Performance of Determination of Metastatic Bone Tumor in Renal Cancer Serum Samples

The details of the serum sample panel (total of 29 cases) used in the present Example are shown in Table 6. All the samples were collected in the same protocol in the Department of Urology at Osaka University Hospital. The samples were provided after obtainment of informed consent and approval by the Clinical Research Review Committee in Osaka University.

**[Table 6]**

| | Cases |
|---|---|
| Renal Cancer (Without Bone Metastasis) | 9 |
| Metastatic Bone Tumor of Renal Cancer | 20 |

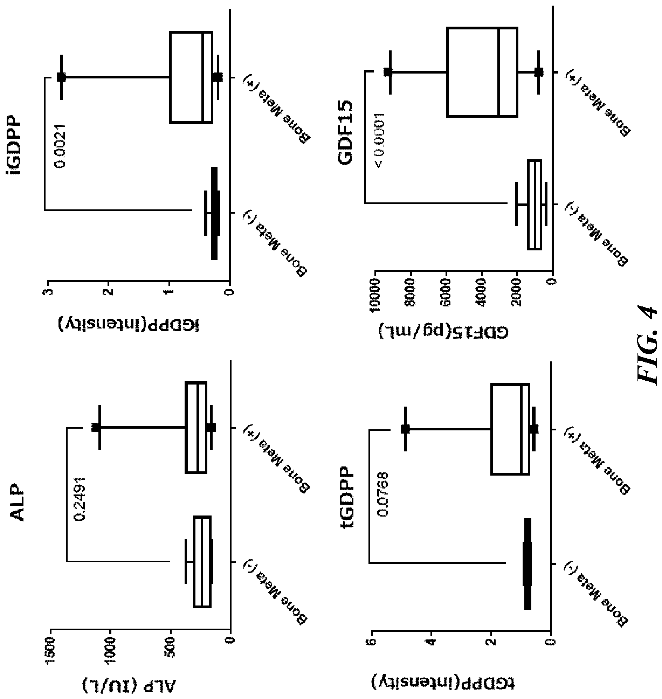

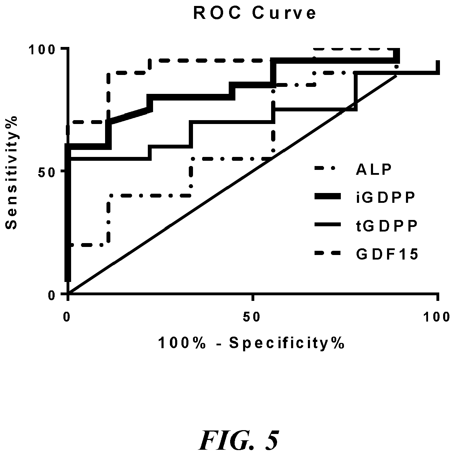

In this examination, ALP, iGDPP, tGDPP, and GDF15 were assayed, and their performances of determination of metastatic bone tumor in renal cancer serum samples were compared. The measured values of iGDPP and tGDPP were calculated using a dedicated assay reagent and a fully automatic enzyme immunoassay apparatus AIA-600II (manufactured by Tosoh Corporation), and the measured value of GDF15 was calculated using a commercially available ELISA kit (R & D Corporation). For ALP, reference to measured values just after blood sampling in medical service under health insurance was performed. Box plots of the various measured values are shown in FIG. 4, ROC analysis is shown in FIG. 5, and significant test results as determined by Mann Whitney's U test and the values of AUC as determined by the ROC analysis are shown in Table 7.

**[Table 7]**

| | | Renal Cancer (Without Bone Metastasis) (9 cases) VS Metastatic Bone Tumor of Renal Cancer (20 cases) | |
|---|---|---|---|
| | | P values of Mann Whitney's U test | Area Under the Curve (AUC) |
| ALP | Serum | 0.249 | 0.639 |
| iGDPP | | 0.002 | 0.847 |
| tGDPP | | 0.077 | 0.711 |
| GDF15 | | < 0.001 | 0.933 |

For ALP, the performance of determination of metastatic bone tumor in renal cancer was shown to be low, as similarly shown in prostate cancer. On the other hand, iGDPP and GDF15 were shown to have the favorable performance of determination in the renal cancer serum samples.

### <Example 5> Performance of Determination of Metastatic Bone Tumor in Lung Cancer and Breast Cancer Plasma Samples

The details of the plasma sample panel (total of 29 cases) used in the present Example are shown in Table 8. The serum samples of healthy individuals were purchased from BioreclamationIVT, and various cancer serum samples were purchased from PROMEDDX. It is clearly described in the documents attached to the products of both companies, that these samples were collected in accordance with the protocols approved by the ethics committee.

**[Table 8]**

| | Cases |
|---|---|
| Normal | 16 |
| Metastatic Bone Tumor of Lung Cancer | 8 |
| Metastatic Bone Tumor of Breast Cancer | 5 |

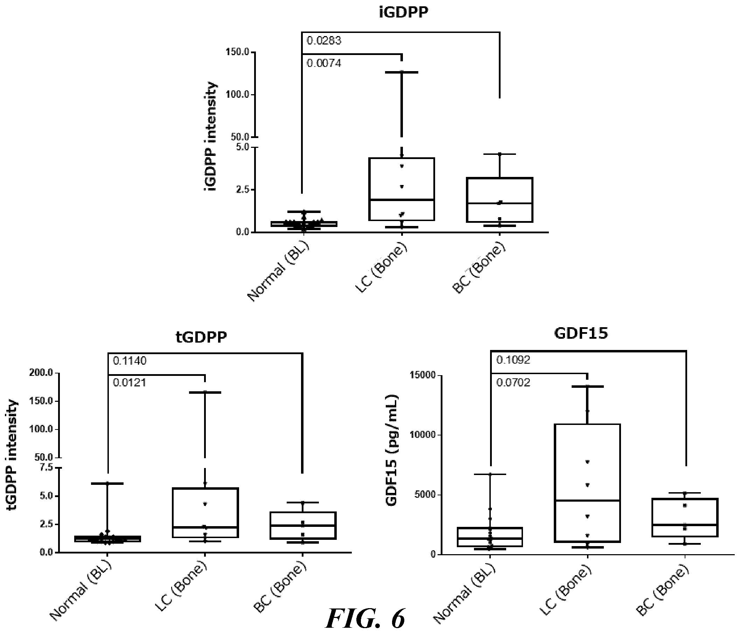

In this examination, iGDPP, tGDPP, and GDF15 were assayed, and their performances of determination of normal samples and metastatic bone tumor of lung cancer or metastatic bone tumor of breast cancer were compared. The measured values of iGDPP and tGDPP were calculated using a dedicated assay reagent and a fully automatic enzyme immunoassay apparatus AIA-600II (manufactured by Tosoh Corporation), and the measured value of GDF15 was calculated using a commercially available ELISA kit (R & D Corporation). Box plots of the various measured values are shown in FIG. 6, and significant test results as determined by Mann Whitney's U test are shown in Table 9.

**[Table 9]**

| | P values of Mann Whitney's U test | |
|---|---|---|
| | Normal (16 cases) VS Metastatic Bone Tumor of Lung Cancer (8 cases) | Normal (16 cases) VS Metastatic Bone Tumor of Breast Cancer (5 cases) |
| iGDPP | 0.0074 | 0.0283 |
| tGDPP | 0.0121 | 0.1140 |
| GDF15 | 0.0702 | 0.1092 |

For the performance of the determination of the metastatic bone tumor of the lung cancer or the metastatic bone tumor of the breast cancer, iGDPP was shown to be the most favorable. The relatively favorable performance of the determination was also observed in GDF15. Increased blood levels in some healthy individuals are considered to result in deterioration of the performance of the determination.

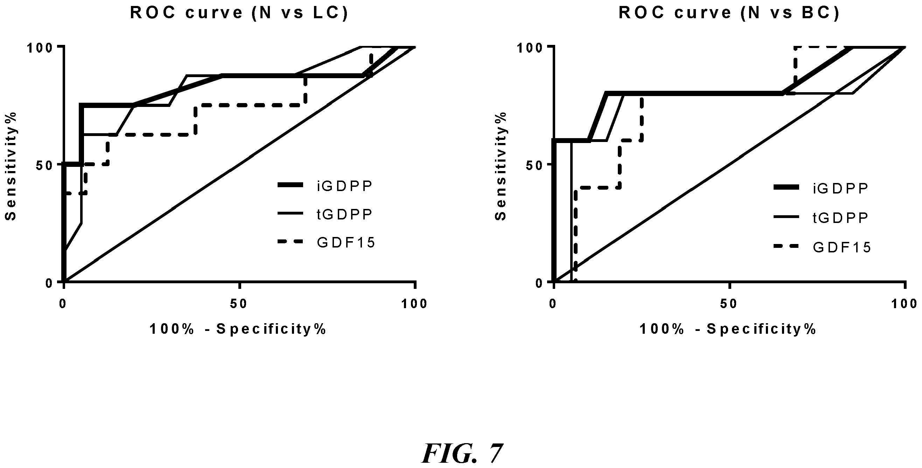

Then, examination of the performance of determination of metastatic bone tumor of lung cancer or metastatic bone tumor of breast cancer was carried out by ROC curve analysis. The analysis results are shown in FIG. 7, and the values of AUC are shown in Table 10.

**[Table 10]**

| | Area Under the Curve (AUC) | |
|---|---|---|
| | Normal (16 cases) VS Metastatic Bone Tumor of Lung Cancer (8 cases) | Normal (16 cases) VS Metastatic Bone Tumor of Breast Cancer (5 cases) |
| iGDPP | 0.8281 | 0.8188 |
| tGDPP | 0.8125 | 0.7438 |
| GDF15 | 0.7344 | 0.7500 |

For the performance of the determination of either the metastatic bone tumor of the lung cancer or the metastatic bone tumor of the breast cancer, iGDPP was shown to be the most favorable.

The above Examples of the present invention showed that GDPP propeptide has the significantly higher performance of determination of metastatic bone tumor than ALP considered to be an indicator for bone metastasis. Further, in the case of using the plasma samples, iGDPP was shown to have the superior performance of determination of metastatic bone tumor to GDF15.

### <Example 6> Performance of Determination of Metastatic Bone Tumor in Serum Samples of Prostate Cancer (Non-CRPC) Which is Not Castration Resistant Prostate Cancer (CRPC)

The details the prostate cancer serum sample panel (total of 40 cases) used in Example 2, classified based on CRPC/non-CRPC, and on the absence/presence of metastatic bone tumor, are shown in Table 11.

**[Table 11]**

| | Cases | |
|---|---|---|
| | CRPC | Non CRPC |
| Absence of Metastatic Bone Tumor | 4 | 18 |
| Presence of Metastatic Bone Tumor | 15 | 3 |

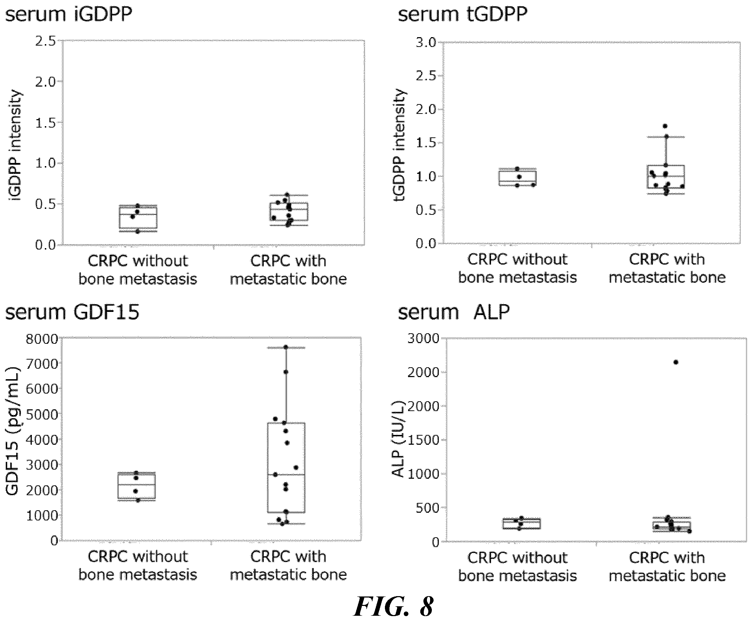

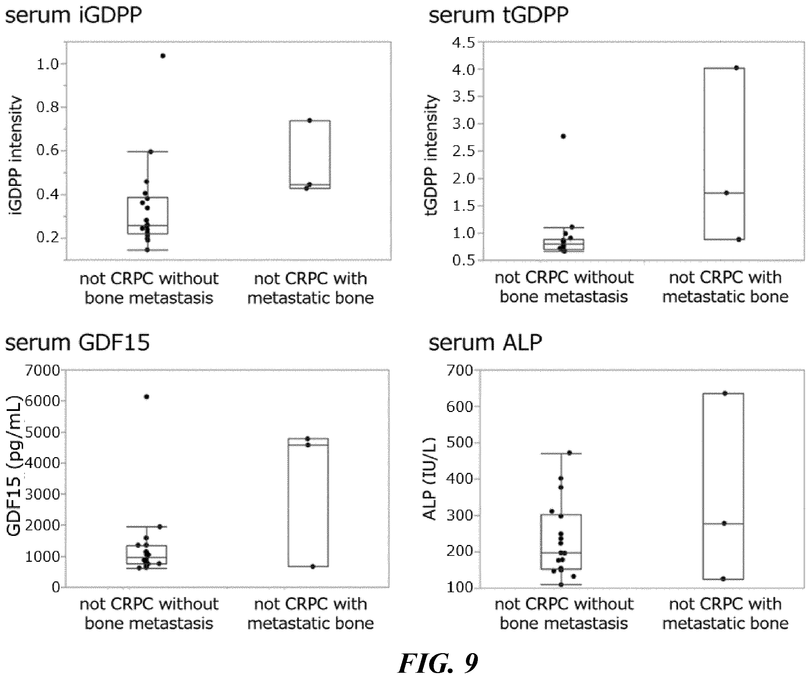

In this examination, iGDPP, tGDPP, GDF15, and alkaline phosphatase (ALP) were assayed, and their performances of determination of metastatic bone tumor in the CRPC group and the non-CRPC group were compared. The measured values of iGDPP and tGDPP were calculated using a dedicated assay reagent and a fully automatic enzyme immunoassay apparatus AIA-600II (manufactured by Tosoh Corporation), and the measured value of GDF15 was calculated using a commercially available ELISA kit (R & D Corporation). For ALP, reference to measured values just after blood sampling in medical service under health insurance was performed. Box plots of the various measured values in the CRPC group are shown in FIG. 8, box plots of the various measured values in the non-CRPC group are shown in FIG. 9, and significant test results as determined by Mann Whitney's U test are shown in Table 12.

**[Table 12]**

| | | P values of Mann Whitney's U test | |
|---|---|---|---|
| | | <CRPC> Absence of Metastatic Bone Tumor (4) VS Presence of Metastatic Bone Tumor (15) | <Non CRPC> Absence of Metastatic Bone Tumor (18) VS Presence of Metastatic Bone Tumor (3) |
| iGDPP | Serum | 0.484 | 0.044 |
| tGDPP | | 0.920 | 0.027 |
| GDF15 | | 0.617 | 0.366 |
| ALP | | 0.424 | 0.615 |

No significant difference was observed in each marker in the CRPC group, and a significant difference was observed each of iGDPP and tGDPP in the non-CRPC group.

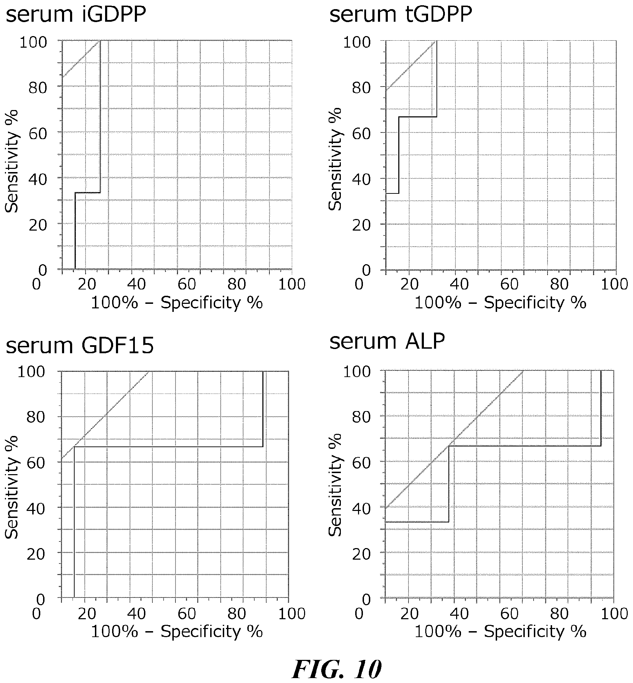

Then, examination of the performance of determination of metastatic bone tumor in the non-CRPC group was carried out by receiver operating characteristic (ROC) curve analysis. The analysis results are shown in FIG. 10, and the values of AUC (Area Under the Curve; the area under the ROC curve) are shown in Table 13.

**[Table 13]**

| | | Area Under the Curve (AUC) |
|---|---|---|
| | | <Non CRPC> Absence of Metastatic Bone Tumor (18) VS Presence of Metastatic Bone Tumor (3) |
| iGDPP | Serum | 0.870 |
| tGDPP | | 0.907 |
| GDF15 | | 0.667 |
| ALP | | 0.593 |

For the performance of determination of the metastatic bone tumor in the non-CRPC group, iGDPP and tGDPP were shown to be superior to the other markers including ALP.

### <Example 7> Performance of Determination of Metastatic Bone Tumor in Serum and Plasma Samples of Lung Cancer

The details of the serum and plasma sample panel (total of 27 cases) used in the present Example are shown in Table 14 (the cases with the plasma samples of the metastatic bone tumor are the same as the cases in Example 5). The samples used were purchased from BioreclamationIVT and PROMEDDX. It is clearly described in the documents attached to the products, that these samples were collected in accordance with the protocols approved by the ethics committee.

**[Table 14]**

| Lung Cancer Cases | Cases | |
|---|---|---|
| | Serum | Plasma |
| Without Metastasis | 4 | 5 |
| Metastasis to Any Site Other Than Bone | 2 | 3 |
| Metastatic Bone Tumor | 5 | 8 |

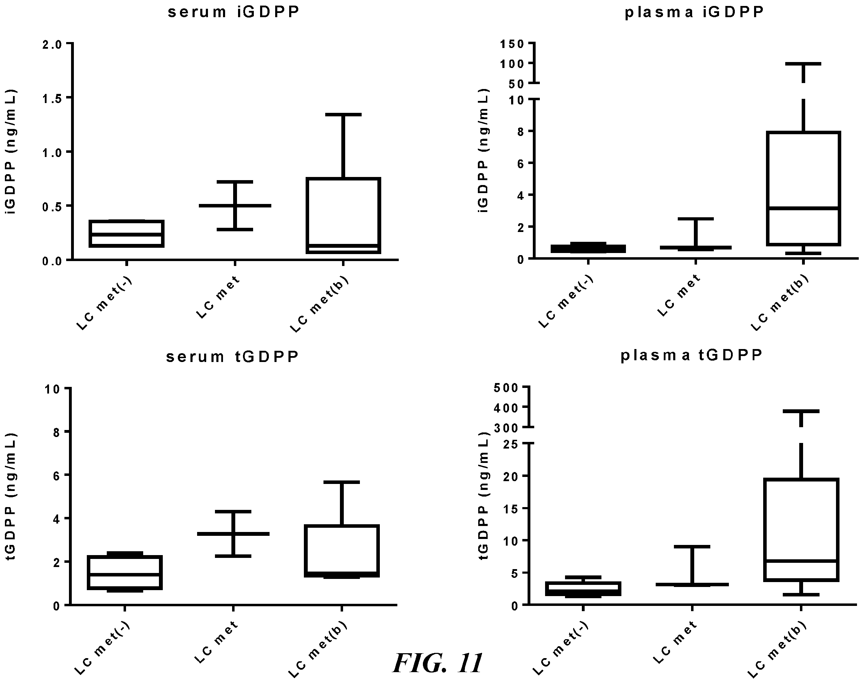

In this examination, iGDPP and tGDPP were assayed, and their performances of determination of metastatic bone tumor in lung cancer were compared. The measured values of iGDPP and tGDPP were calculated using a dedicated assay reagent and a fully automatic enzyme immunoassay apparatus AIA-600II (manufactured by Tosoh Corporation). Box plots of the various measured values are shown in FIG. 11, and significant test results as determined by Mann Whitney' s U test are shown in Table 15.

**[Table 15]**

| | P values of Mann Whitney's U test | |
|---|---|---|
| | <Lung Cancer Serum> Absence of Metastatic Bone Tumor (6) Vs Presence of Metastatic Bone Tumor (5) | <Lung Cancer Plasma> Absence of Metastatic Bone Tumor (8) Vs Presence of Metastatic Bone Tumor (8) |
| iGDPP | 0.273 | 0.036 |
| tGDPP | 0.855 | 0.036 |

No significant difference was observed in the serum samples, while a significant difference was observed in each of iGDPP and tGDPP in the plasma samples. The cause of the determination performance varying depending on the serum and the plasma is considered to be an influence due to stability in a sample, such as degradation by protease, although it is impossible to generalize about the cause because evaluation with the serum/plasma pair samples of the same case was not carried out.

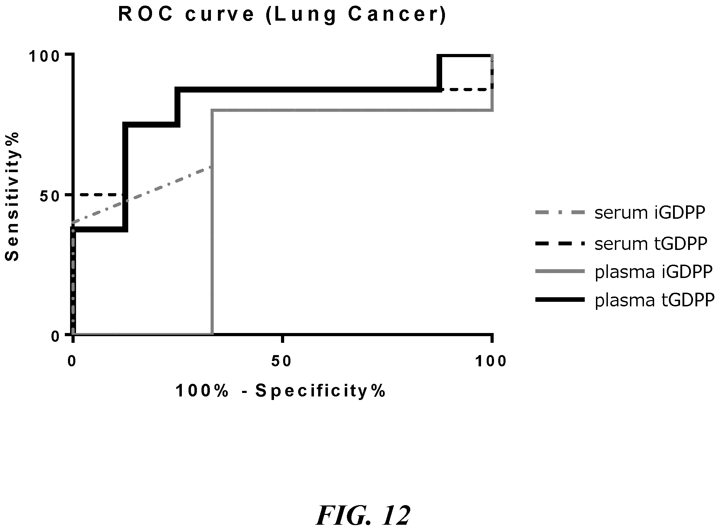

Then, examination of the performance of determination of the metastatic bone tumor in the lung cancer was carried out by receiver operating characteristic (ROC) curve analysis. The analysis results are shown in FIG. 12, and the values of AUC (Area Under the Curve; the area under the ROC curve) are shown in Table 16.

**[Table 16]**

| | Area Under the Curve (AUC) | |
|---|---|---|
| | <Lung Cancer Serum> Absence of Metastatic Bone Tumor (6) Vs Presence of Metastatic Bone Tumor (5) | <Lung Cancer Plasma> Absence of Metastatic Bone Tumor (8) Vs Presence of Metastatic Bone Tumor (8) |
| iGDPP | 0.700 | 0.813 |
| tGDPP | 0.533 | 0.813 |

The assay of iGDPP or tGDPP in the plasma samples showed that iGDPP or tGDPP has the excellent performance of the determination of the metastatic bone tumor of the lung cancer.

### <Example 8> Performance of Determination of Metastatic Bone Tumor in Serum and Plasma Samples of Breast Cancer

The details of the serum and plasma sample panel (total of 28 cases) used in the present Example are shown in Table 17 (the cases with the plasma samples of the metastatic bone tumor are the same as the cases in Example 5). The samples used were purchased from BioreclamationIVT and PROMEDDX. It is clearly described in the documents attached to the products, that these samples were collected in accordance with the protocols approved by the ethics committee.

**[Table 17]**

| Breast Cancer cases | Cases | |
|---|---|---|
| | Serum | Plasma |
| Without Bone Metastasis | 5 | 5 |
| Metastasis To Any Site Other Than Bone | 3 | 4 |
| Metastatic Bone Tumor | 5 | 6 |

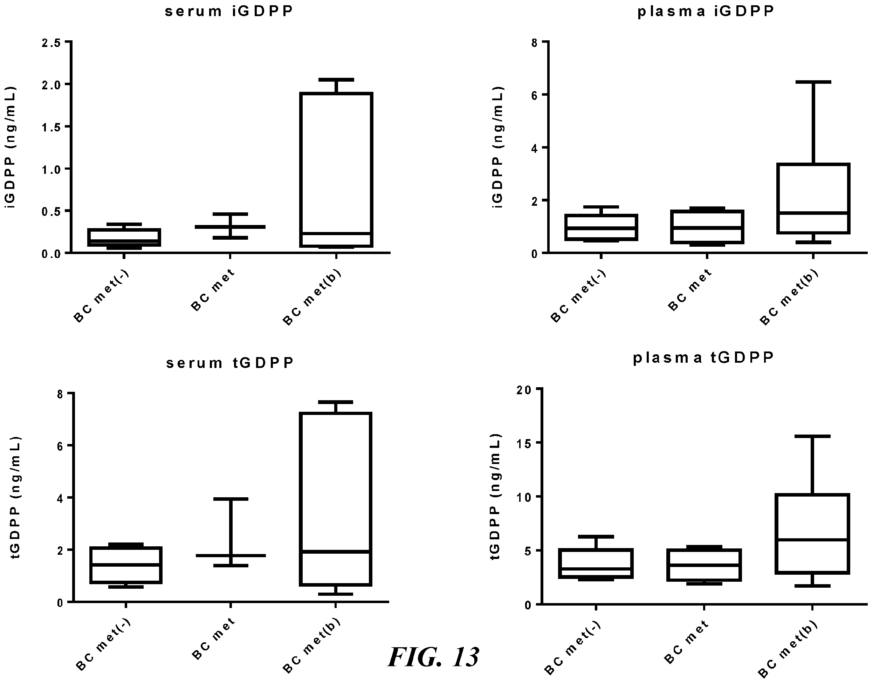

In this examination, iGDPP and tGDPP were assayed, and their performances of determination of metastatic bone tumor in breast cancer were compared. The measured values of iGDPP and tGDPP were calculated using a dedicated assay reagent and a fully automatic enzyme immunoassay apparatus AIA-600II (manufactured by Tosoh Corporation). Box plots of the various measured values are shown in FIG. 13, and significant test results as determined by Mann Whitney' s U test are shown in Table 18.

**[Table 18]**

| | P values of Mann Whitney's U test | |
|---|---|---|
| | <Breast Cancer Serum> Absence of Metastatic Bone Tumor (8) VS Presence Of Metastatic Bone Tumor (5) | <Breast Cancer Plasma> Absence of Metastatic Bone Tumor (9) VS Presence Of Metastatic Bone Tumor (6) |
| iGDPP | 0.661 | 0.223 |
| tGDPP | 0.661 | 0.157 |

As for the metastatic bone tumor of the breast cancer, there were some cases with the increased concentration of GDPP; however, since the number of the cases was small, no statistically significant difference was observed.

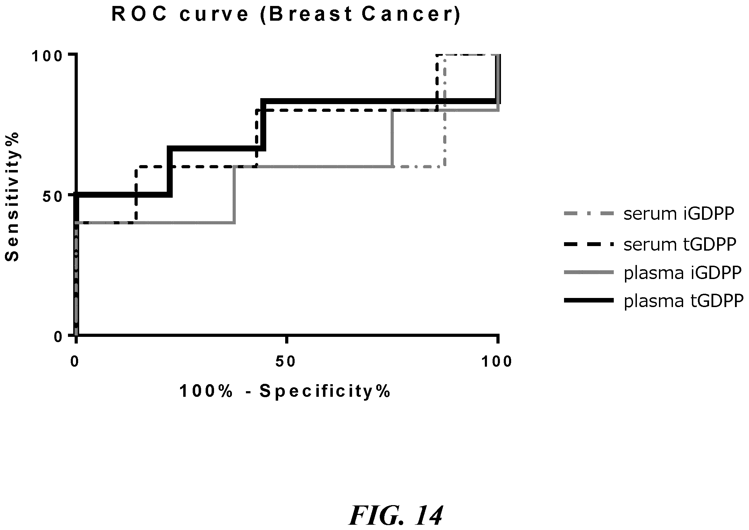

Then, examination of the performance of determination of the metastatic bone tumor in the breast cancer was carried out by receiver operating characteristic (ROC) curve analysis. The analysis results are shown in FIG. 14, and the values of AUC (Area Under the Curve; the area under the ROC curve) are shown in Table 19.

**[Table 19]**

| | Area Under the Curve (AUC) | |
|---|---|---|
| | <Breast Cancer Serum> Absence of Metastatic Bone Tumor (8) VS Presence of Metastatic Bone Tumor (5) | <Breast Cancer Plasma> Absence of Metastatic Bone Tumor (9) VS Presence of Metastatic Bone Tumor (6) |
| iGDPP | 0.575 | 0.714 |
| tGDPP | 0.575 | 0.722 |

As for the determination of the metastatic bone tumor in the breast cancer, slightly favorable performance was shown in the assay of tGDPP in the plasma samples.

### <Example 9> Performance of Determination of Metastatic Bone Tumor in Plasma Samples of Various Types of Cancer

The details of the plasma sample panel (total of 34 cases) used in the present Example are shown in Table 20. The samples used were purchased from BioreclamationIVT and PROMEDDX. It is clearly described in the documents attached to the products, that these samples were collected in accordance with the protocols approved by the ethics committee.

**[Table 20]**

| | Cases |
|---|---|
| Normal | 5 |
| Thyroid Cancer Metastatic Bone Tumor | 2 |
| Pancreatic Cancer Metastatic Bone Tumor | 1 |
| Bladder Cancer Metastatic Bone Tumor | 1 |
| Colon Cancer Metastatic Bone Tumor | 6 |
| Melanoma Metastatic Bone Tumor | 4 |
| Myeloma Metastatic Bone Tumor | 10 |
| Lymphoma Metastatic Bone Tumor | 5 |

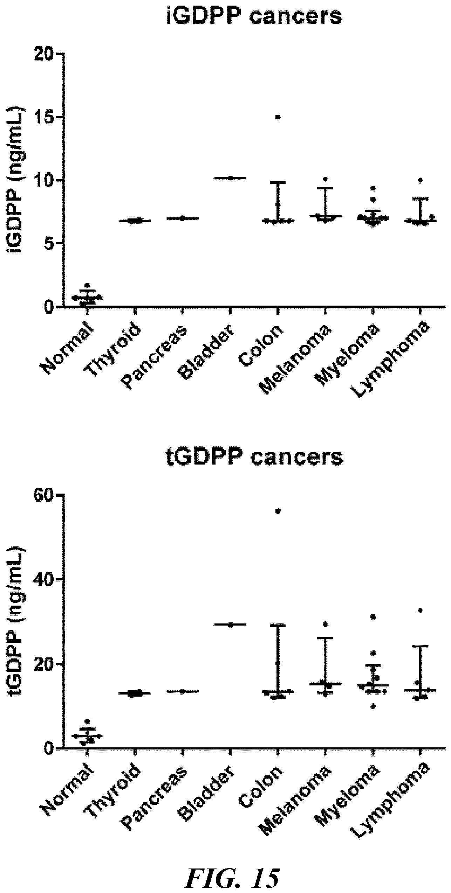

In this examination, iGDPP and tGDPP were assayed, and their performances of determination of metastatic bone tumor in various types of cancer were compared. The measured values of iGDPP and tGDPP were calculated using a dedicated assay reagent and a fully automatic enzyme immunoassay apparatus AIA-600II (manufactured by Tosoh Corporation). Box plots of the various measured values are shown in FIG. 15.

The numbers of cases with some types of cancer were small, and it was thus impossible to sufficiently evaluate a significance test; however, since the blood levels of iGDPP and tGDPP were definitely increased as compared to the normal samples, it was shown that it was possible to determine the metastatic bone tumor in the various types of cancer.

### INDUSTRIAL APPLICABILITY

The present invention provides a method and a reagent which enable detection of bone metastasis of cancer (excluding CRPC) . The above described method and reagent enable a simple and highly accurate detection, by blood diagnosis or the like, of the presence or absence of bone metastasis of cancer (excluding CRPC) which has been difficult to be determined by a conventional marker. As a result, they allow for simple detection of bone metastasis of cancer (excluding CRPC), making it possible to select treatment methods and to determine therapeutic effects, and thus are extremely useful industrially.

## Claims

1. A method for detecting bone metastasis of cancer (excluding castration resistant prostate cancer), the method comprising measuring an intact growth and differentiation factor 15 (GDF15) propeptide level in a sample.

2. A method for detecting bone metastasis of cancer (excluding castration resistant prostate cancer), the method comprising measuring a GDF15 propeptide fragment level in a sample.

3. A method for detecting bone metastasis of cancer (excluding castration resistant prostate cancer), the method comprising measuring a total of an intact GDF15 propeptide level and a GDF15 propeptide fragment level in a sample.

4. The method according to claim 2 or 3, wherein the GDF15 propeptide fragment(s) comprise(s) the following GDF15 propeptide fragment(s) (A) and/or (B):
(A) a GDF15 propeptide fragment having the following properties:
contains an amino acid sequence from the lysine at the 58th residue to at least the aspartic acid at the 167th residue in the GDF15 amino acid sequence of SEQ ID NO:2, or a sequence having an identity of not less than 80% thereto; and
(B) a GDF15 propeptide fragment having the following properties:
contains an amino acid sequence from the glutamic acid at the 74th residue to at least the aspartic acid at the 167th residue in the GDF15 amino acid sequence of SEQ ID NO:2, or a sequence having an identity of not less than 80% thereto.

5. The method according to any one of claims 1 to 4, wherein bone metastasis of prostate cancer other than castration resistant prostate cancer, renal cancer, lung cancer, breast cancer, thyroid cancer, pancreatic cancer, bladder cancer, colon cancer, melanoma, myeloma, or lymphoma is detected.

6. The method according to any one of claims 1 to 5, wherein the measurement is carried out by an antigen-antibody reaction using an antibody that recognizes GDF15 propeptide.

7. The method according to any one of claims 1 to 5, wherein the measurement is carried out using mass spectrometry.

8. A reagent for detecting bone metastasis of cancer (excluding castration resistant prostate cancer), the reagent comprising an antibody that specifically recognizes GDF15 propeptide.


SEQUENCE LISTING

<110>  Osaka university
       TOSOH corporation

<120>
\202ª\202ñ\202Ì\215\234\223]\210Ú\202ð\214\237\217o\202•\202é\225û\226@\213y
\202Ñ\214\237\217o\216\216\226ò

<130>  2190137-1712

<150>  JP2019-211488
<151>  2019-11-22

<160>  2

<170>  PatentIn version 3.5


<210>  1
<211>  1220
<212>  DNA
<213>  Homo sapiens


<220>
<221>  CDS
<222>  (33)..(956)

<400>  1
agtcccagct cagagccgca acctgcacag cc atg ccc ggg caa gaa ctc agg        53
                                    Met Pro Gly Gln Glu Leu Arg
                                    1               5

acg gtg aat ggc tct cag atg ctc ctg gtg ttg ctg gtg ctc tcg tgg        101
Thr Val Asn Gly Ser Gln Met Leu Leu Val Leu Leu Val Leu Ser Trp
        10                  15                  20

ctg ccg cat ggg ggc gcc ctg tct ctg gcc gag gcg agc cgc gca agt        149
Leu Pro His Gly Gly Ala Leu Ser Leu Ala Glu Ala Ser Arg Ala Ser
    25                  30                  35

ttc ccg gga ccc tca gag ttg cac tcc gaa gac tcc aga ttc cga gag        197
Phe Pro Gly Pro Ser Glu Leu His Ser Glu Asp Ser Arg Phe Arg Glu
40                  45                  50                  55

ttg cgg aaa cgc tac gag gac ctg cta acc agg ctg cgg gcc aac cag        245
Leu Arg Lys Arg Tyr Glu Asp Leu Leu Thr Arg Leu Arg Ala Asn Gln
                60                  65                  70

agc tgg gaa gat tcg aac acc gac ctc gtc ccg gcc cct gca gtc cgg        293
Ser Trp Glu Asp Ser Asn Thr Asp Leu Val Pro Ala Pro Ala Val Arg
            75                  80                  85

ata ctc acg cca gaa gtg cgg ctg gga tcc ggc ggc cac ctg cac ctg        341
Ile Leu Thr Pro Glu Val Arg Leu Gly Ser Gly Gly His Leu His Leu
        90                  95                  100

cgt atc tct cgg gcc gcc ctt ccc gag ggg ctc ccc gag gcc tcc cgc        389
Arg Ile Ser Arg Ala Ala Leu Pro Glu Gly Leu Pro Glu Ala Ser Arg
    105                 110                 115

ctt cac cgg gct ctg ttc cgg ctg tcc ccg acg gcg tca agg tcg tgg        437

Leu His Arg Ala Leu Phe Arg Leu Ser Pro Thr Ala Ser Arg Ser Trp
120                 125                 130                 135

gac gtg aca cga ccg ctg cgg cgt cag ctc agc ctt gca aga ccc cag      485
Asp Val Thr Arg Pro Leu Arg Arg Gln Leu Ser Leu Ala Arg Pro Gln
                140                 145                 150

gcg ccc gcg ctg cac ctg cga ctg tcg ccg ccg ccg tcg cag tcg gac      533
Ala Pro Ala Leu His Leu Arg Leu Ser Pro Pro Pro Ser Gln Ser Asp
            155                 160                 165

caa ctg ctg gca gaa tct tcg tcc gca cgg ccc cag ctg gag ttg cac      581
Gln Leu Leu Ala Glu Ser Ser Ser Ala Arg Pro Gln Leu Glu Leu His
        170                 175                 180

ttg cgg ccg caa gcc gcc agg ggg cgc cgc aga gcg cgt gcg cgc aac      629
Leu Arg Pro Gln Ala Ala Arg Gly Arg Arg Arg Ala Arg Ala Arg Asn
    185                 190                 195

ggg gac cac tgt ccg ctc ggg ccc ggg cgt tgc tgc cgt ctg cac acg      677
Gly Asp His Cys Pro Leu Gly Pro Gly Arg Cys Cys Arg Leu His Thr
200                 205                 210                 215

gtc cgc gcg tcg ctg gaa gac ctg ggc tgg gcc gat tgg gtg ctg tcg      725
Val Arg Ala Ser Leu Glu Asp Leu Gly Trp Ala Asp Trp Val Leu Ser
                220                 225                 230

cca cgg gag gtg caa gtg acc atg tgc atc ggc gcg tgc ccg agc cag      773
Pro Arg Glu Val Gln Val Thr Met Cys Ile Gly Ala Cys Pro Ser Gln
            235                 240                 245

ttc cgg gcg gca aac atg cac gcg cag atc aag acg agc ctg cac cgc      821
Phe Arg Ala Ala Asn Met His Ala Gln Ile Lys Thr Ser Leu His Arg
        250                 255                 260

ctg aag ccc gac acg gtg cca gcg ccc tgc tgc gtg ccc gcc agc tac      869
Leu Lys Pro Asp Thr Val Pro Ala Pro Cys Cys Val Pro Ala Ser Tyr
    265                 270                 275

aat ccc atg gtg ctc att caa aag acc gac acc ggg gtg tcg ctc cag      917
Asn Pro Met Val Leu Ile Gln Lys Thr Asp Thr Gly Val Ser Leu Gln
280                 285                 290                 295

acc tat gat gac ttg tta gcc aaa gac tgc cac tgc ata tgagcagtcc      966
Thr Tyr Asp Asp Leu Leu Ala Lys Asp Cys His Cys Ile
                300                 305

tggtccttcc actgtgcacc tgcgcggagg acgcgacctc agttgtcctg ccctgtggaa      1026

tgggctcaag gttcctgaga cacccgattc ctgcccaaac agctgtattt atataagtct      1086

gttatttatt attaatttat tggggtgacc ttcttgggga ctcgggggct ggtctgatgg      1146

aactgtgtat ttatttaaaa ctctggtgat aaaaataaag ctgtctgaac tgttaaaaaa      1206

aaaaaaaaaa aaaa                                                        1220

<210>  2
<211>  308
<212>  PRT
<213>  Homo sapiens

<400>  2

Met Pro Gly Gln Glu Leu Arg Thr Val Asn Gly Ser Gln Met Leu Leu
1               5                   10                  15

Val Leu Leu Val Leu Ser Trp Leu Pro His Gly Gly Ala Leu Ser Leu
            20                  25                  30

Ala Glu Ala Ser Arg Ala Ser Phe Pro Gly Pro Ser Glu Leu His Ser
        35                  40                  45

Glu Asp Ser Arg Phe Arg Glu Leu Arg Lys Arg Tyr Glu Asp Leu Leu
    50                  55                  60

Thr Arg Leu Arg Ala Asn Gln Ser Trp Glu Asp Ser Asn Thr Asp Leu
65                  70                  75                  80

Val Pro Ala Pro Ala Val Arg Ile Leu Thr Pro Glu Val Arg Leu Gly
                85                  90                  95

Ser Gly Gly His Leu His Leu Arg Ile Ser Arg Ala Ala Leu Pro Glu
            100                 105                 110

Gly Leu Pro Glu Ala Ser Arg Leu His Arg Ala Leu Phe Arg Leu Ser
        115                 120                 125

Pro Thr Ala Ser Arg Ser Trp Asp Val Thr Arg Pro Leu Arg Arg Gln
    130                 135                 140

Leu Ser Leu Ala Arg Pro Gln Ala Pro Ala Leu His Leu Arg Leu Ser
145                 150                 155                 160

Pro Pro Pro Ser Gln Ser Asp Gln Leu Leu Ala Glu Ser Ser Ser Ala
                165                 170                 175

Arg Pro Gln Leu Glu Leu His Leu Arg Pro Gln Ala Ala Arg Gly Arg
            180                 185                 190

Arg Arg Ala Arg Ala Arg Asn Gly Asp His Cys Pro Leu Gly Pro Gly
        195                 200                 205

Arg Cys Cys Arg Leu His Thr Val Arg Ala Ser Leu Glu Asp Leu Gly
    210                 215                 220

Trp Ala Asp Trp Val Leu Ser Pro Arg Glu Val Gln Val Thr Met Cys
225                 230                 235                 240

Ile Gly Ala Cys Pro Ser Gln Phe Arg Ala Ala Asn Met His Ala Gln
                245                 250                 255

Ile Lys Thr Ser Leu His Arg Leu Lys Pro Asp Thr Val Pro Ala Pro
            260                 265                 270

Cys Cys Val Pro Ala Ser Tyr Asn Pro Met Val Leu Ile Gln Lys Thr
        275                 280                 285

Asp Thr Gly Val Ser Leu Gln Thr Tyr Asp Asp Leu Leu Ala Lys Asp
    290                 295                 300

Cys His Cys Ile
305